# EUROPÄISCHE PATENTSCHRIFT

(11) **EP 0 500 844 B1**
(45) Veröffentlichungstag und Bekanntmachung des Hinweises auf die Patenterteilung: **21.08.1996**
(21) Anmeldenummer: 91914910.4
(22) Anmeldetag: 06.09.1991
(51) Int. Cl.: A61K 38/00, A61K 39/00, C12N 5/00, A01N 1/00

(54) **ARZNEIMITTEL ENTHALTEND CD14**
DRUG CONTAINING CD14
MEDICAMENT CONTENANT DU CD14

(30) Priorität: 14.09.1990 DE 4029227
(43) Veröffentlichungstag der Anmeldung: 02.09.1992
(73) Patentinhaber: IMTOX PRIVATINSTITUT FÜR IMMUNBIOLOGISCHE FORSCHUNG GMBH, D-13355 Berlin (DE)
(72) Erfinder: SCHÜTT, Christine, D-2200 Greifswald (DE); KUNZE, Rudolf, D-1000 Berlin 65 (DE)
(74) Vertreter: Werner, Hans-Karsten, Dr.Dipl.-Chem.
(86) Internationale Anmeldenummer: EP9101697
(87) Internationale Veröffentlichungsnummer: WO9204908

(56) Entgegenhaltungen:
- EP-A- 0 330 191
- WO-A-86/06279
- WO-A-91/01639
- WO-A-91/11464
- Dialog Inromation Services, file 155, Medline, accession no. 06613060; Haziot A et al.: "The monocyte differentiation antigen, CD 14, is anchored to the cell membrane by phosphatidylinositol linkage"
- Dialog Information Services, File 155, Medline, accession No. 07082684; Bazil V et al.: "Structural relationship between the soluble and membrane-bound forms of human monocyte surface glycoprotein CD 14"
- Science, Vol. 239, 1988; Sanna M. Goyert et al.: "The CD14 Monocyte Differenttiation Antigen Maps to a Region Encoding Growth Factors and Receptors", page 497-500
- Science, vol 249, sep. 1990; Samuel D. Wright et al.: "CD 14, a Receptor for complexes of Lipopolysaccharide (LPS) and LPS Binding Protein", page 1431-1433
- J. Trauma, Vol. 30, no. 12, 1990; Richard J. Ulevitch et al.: "A New Model of Macrophage Stimulation by Bacterial Lipopolysaccharide", page 189-192, Proceed, NIH conference 21-23 June 1990 Advances in Understanding Trauma and Burn Injury
- Dialog Information Services, File 155; Weisman HF et al.: "Soluble human complement receptor type 1: in vivo inhibitor of complement suppressing post-ischemic myocardial inflammation and necrosis"

## Beschreibung

Gegenstand der vorliegenden Erfindung ist ein Arzneimittel enthaltend CD14, welches aus dem Serum oder anderen Körperflüssigkeiten von Mensch und Tier isoliert wurde, sowie die Verwendung von CD14 zur Herstellung von Arzneimitteln zur Therapie oder Prophylaxe von Erkrankungen, insbesondere zur Therapie einer Sepsis. Darüber hinaus ist Gegenstand der vorliegenden Erfindung die Verwendung von CD14 als Endotoxine neutralisierendes Prinzip.

CD14 (Cluster of Differentiation) ist ein im Menschen vorkommendes, glycosyliertes Protein. Es charakterisiert die sogenannten Monozyten, die unter anderem im peripheren Blut des Menschen vorkommen. Diese Molekül kann auf der Zelloberfläche mit modernen immundiagnostischen Methoden und mittels Verwendung von monoklonalen Antikörpern nachgewiesen werden. Aus der wissenschaftlichen Literatur ist weiterhin bekannt, daß CD14 nicht nur auf Oberflächen von Monozyten und aktivierten neutrophilen Granulozyten vorkommt, sondern daß es in derselben oder in einer geringfügig abgewandelten molekularen Form als lösliches CD14 (sCD14) im Serum und anderen Körperflüssigkeiten des Menschen nachgewiesen werden kann. Es ist darüber hinaus bekannt, daß dieses Molekül im Serum bei bestimmten Krankheitsbildern entweder in einem erhöhten oder aber in einem erniedrigten Spiegel, in jedem Fall außerhalb der normalen Toleranzgrenze, auftritt.

Der endotoxische Schock ist ein großes klinisches Problem. Der endotoxische Schock kann im Verlauf einer Sepsis aufgrund verschiedener Ursachen auftreten. Monokine wie Tumornekrosefaktor (TNF-alpha), Interleukin-1, Interleukin-6 oder PAF und freie Sauerstoffradikale spielen eine Schlüsselrolle in der Pathophysiologie der Traumata, in deren Verlauf Sepsis und Schock auftreten.

Endotoxine sind potente Monozyten- und Endothelzellaktivatoren. Durch die nachgeschalteten weiteren humoralen und zellulären Aktivierungsprozesse kommt es zu Endothel- und Gewebsläsionen. Nach diesen überschießenden Immunreaktionen ist die lokale Immunabwehr und auch -barriere nicht mehr voll funktionsfähig.

Aus A. Haziot et al., J. Immunol. 141 (1988), Seiten 547 bis 552, ist die CD14-Sequenz bekannt. Darüber hinaus wird in dieser Veröffentlichung festgestellt, daß CD14 über Phosphatidylinositol-Bindungen an die Zellmembran koppelt. Weiterhin ist aus S. M. Goyert et al., Scienes, 239 (1988), Seiten 497 bis 500, bekannt, daß CD14 5 N-Glycosylierungsstellen aufweist.

Lösliches CD14 (sCD14) hat ein Molekulargewicht von etwa 55.000 Dalton. Es läßt sich mittels bekannter Methoden aus menschlichem Serum bzw. Urin (wenn bei bestimmten Krankheitsbildern ein hoher CD14-Gehalt festgestellt wird) gewinnen. Im folgenden ist eine entsprechende Präparation beispielhaft angeführt.

Material: Urin eines Patienten mit sekundärer Amyloidose
1. Ammoniumsulfat-Fällung (516 g/l); waschen; lyophilisieren.
2. Affinitätschromatographie (mittels eines monoklonalen Antikörpers gegen CD14, der wiederum an CNBr aktivierte Sepharose 4B (5mg AK/ml Gel) gekoppelt ist); Waschen mit PBS (Phosphate Buffered Saline), Elution gebundener Proteine mit 0,1 M Äthanolamin, Aufnahme in 1 M Phosphatpuffer (pH 7,2), Titration mit Essigsäure auf pH 7,0.
3. Wiederholung der Affinitätschromatographie (s. 2).
4. Adsorption an Schwein-Anti-Human-Ig-Antikörper.
5. Dialyse gegen 10 mM Ammoniumhydrogencarbonat-Lösung.
6. Lyophilisierung.

Weiterhin ist bei Goyert et al. (siehe oben) die Herstellung einer cDNA, die für das CD14 codiert, beschrieben. Demnach ist also auch die Herstellung von CD14 bzw. sCD14 auf gentechnologischem Weg möglich.

Die EP-A-0 330 191 beschreibt eine schnelle Immunoselektions-Klonierungs-Methode. Dieses Verfahren wurde bei der Klonierung von Zelloberflächenantigenen von menschlischen Lymphozyten verifiziert. Die EP-A-0 330 191 erwähnt die Verwendung gentechnologisch hergestellter CDMoleküle als therapeutische und diagnostische Werkzeuge für verschiedene Zwecke. Dort wird auch beschrieben, daß aktive Fragmente der gentechnologisch hergestellten CD-Moleküle zu diesen Zwecken eingesetzt werden könnten. Betont wird dabei ausdrücklich die Verwendung von rekombinanten CD-Molekülen und nicht etwa aus natürlichen Quellen isolierte CD-Moleküle.

Aufgabe der vorliegenden Erfindung ist die Bereitstellung eines Arzneimittels zur Therapie oder Prophylaxe von viralen oder bakteriellen Infektionen, insbesondere solchen, die im Zusammenhang mit Sepsis und dem endotoxischen Schock stehen. Das wirksame Prinzip dieses Arzneimittels sollte auch eine hohe Endotoxin-Bindungskapazität aufweisen und in entsprechend zubereiteter Form in präventiver Weise eingesetzt werden können, um beispielsweise iatrogene Einwirkungen in Form von Einschleppung von Endotoxinen während einer Operation oder einer Organverpflanzung zu verhindern.

Diese Aufgabe wird überraschend gelöst durch ein Arzneimittel, enthaltend CD14, welches aus dem Serum oder anderen Körperflüssigkeiten von Mensch und Tier isoliert wurde. Diese können z.B. humane Fibroblastenzellkulturen sein. Die Herstellung synthetischer Peptide, denen die CD14-Aminosäuresequenz oder entsprechende funktionsrelevante Teile davon zugrunde liegen, ist ebenfalls eine Möglichkeit der Bereitstellung von CD14, insbesondere löslichem CD14.

Überraschend war die hohe Bindungskapazität des CD14, insbesondere des löslichen CD14 (sCD14), für Endotoxine.

Eine bevorzugte Ausführungsform des erfindungsgemäßen Arzneimittels enthält lösliches CD14 (sCD14). Dieses Mittel kann auch pharmakologisch wirksame Teilsequenzen aus CD14 oder CD14-Derivaten, gegebenenfalls neben CD14 enthalten. Unter pharmakologisch wirksamen Teilsequenzen aus CD14 oder CD14-Derivaten wird in dieser Anmeldung verstanden, daß noch Strukturbestandteile vorhanden sind, die mit einem spezifischen CD14-Antikörper reagieren können. Vorzugsweise weist das CD14 deshalb ein Molekulargewicht von 45.000 bis 55.000 Dalton auf. Von den verschiedenen dem Fachmann selbstverständlichen CD14-Derivaten werden vorzugsweise die glycosylierten eingesetzt.

In einer weiteren Ausgestaltung enthält das erfindungsgemäße Arzneimittel trägergebundenes CD14 und/oder CD14-Derivate. Das erfindungsgemäße Arzneimittel enthält vorzugsweise auch Immunglobuline oder Immunglobulinfraktionen gekoppeltes CD14 und/oder CD14-Derivate. Diese enthalten in Abhängigkeit von der Herstellungsmethode keinen oder einen modifizierten Zuckerrest.

Erfindungsgemäß kann CD14 zur Herstellung eines Arzneimittels verwendet werden, das entweder Endotoxine oder die durch Endotoxine verursachten Veränderungen neutralisiert.

Weiterer Gegenstand ist eine entsprechende Verwendung von CD14 zur Therapie oder Prophylaxe von viralen oder bakteriellen Infektionen. Die erfindungsgemäße Verwendung schließt auch Arzneimittel zur Therapie oder Prophylaxe von Multipler Sklerose, sowie anderer Autoimmun- und auch Infektionserkrankungen, insbesondere solche, bei denen eine sekundäre Immundefizienz auftreten kann, ein. Die sekundäre Immundefizienz tritt beispielsweise infolge von Radio- oder Chemotherapie auf. Sie kann auch altersbedingt sein.

Gegenstand der vorliegenden Erfindung ist weiterhin die Verwendung von CD14 zur Herstellung eines Arzneimittels zur Therapie von Verbrennungen.

Weiterhin ist die Verwendung von CD14 zur Herstellung eines Arzneimittels zur Therapie einer Sepsis und des endotoxischen Schocks Gegenstand der vorliegenden Erfindung.

Das erfindungsgemäße Arzneimittel kann Verwendung finden zur Hemmung der Freisetzung von Mediatoren (z.B. des Tumornekrosefaktors, Interleukin-1, Komplement), die durch Endotoxine induziert werden. CD14, insbesondere lösliches sCD14, kann zur Herstellung eines Arzneimittels zur Behandlung von Tumoren mittels in vitro hergestellter Lymphokin-aktivierter-Killerzellen verwendet werden (LAK-Therapie). Bei dieser Therapie werden Lymphozyten und Monozyten aus dem peripheren Blut von Krebspatienten isoliert und über längere Zeit in Zellkultur gezüchtet. Diese Zellen werden nach ihrer Vermehrung und Aktivierung wieder in die Blutbahn des Spenders zurückgegeben, wo sie nunmehr Krebszellen angreifen. Dabei ist es ein Problem, die außerhalb des menschlichen Körpers hergestellten Zellen möglichst endotoxinfrei zu erhalten. CD14 wirkt hier als Endotoxin Präventionsmittel.

In ähnlichem Sinne wird CD14, insbesondere lösliches CD14, als Zusatz zu Materialien, wie Kulturmedien, Zu sätze, Seren, Plastikmaterialien, wie sie in klinischen und biologischen Laboratorien zur Anzüchtung und Aufbewahrung von Zellkulturen verwendet werden, eingesetzt.

Durch die Verwendung von CD14 wird das Endotoxin quasi neutralisiert, so daß auch Zellen, die nur in endotoxinfreiem Milieu kultivierbar sind, gezüchtet werden können.

Darüber hinaus ist das CD14, insbesondere lösliches CD14, zur Verwendung als Zusatz in Organspüllösungen (z.B. für die Dialyse), Perfusionslösungen oder sonstigen Lösungen zur Aufbewahrung von Organ- oder Gewebetransplantaten geeignet.

Die Verwendung von gentechnologisch hergestellten Arzneimitteln ist problematisch, da eine Endotoxinfreiheit der so hergestellten Arzneimittel garantiert werden muß. Die Verwendung von CD14, insbesondere löslichem CD14, als Zusatz zu Lösungen oder Applikationsformen von rekombinant hergestellten Arzneimitteln eliminiert das Erfordernis, die gentechnisch hergestellten Arzneimittel in aufwendiger Weise von Endotoxinen zu befreien.

## Patentansprüche

1. Arzneimittel enthaltend CD14, welches aus dem Serum oder anderen Körperflüssigkeiten von Mensch und Tier isoliert wurde.

2. Arzneimittel gemäß Anspruch 1, dadurch gekennzeichnet, daß es lösliches CD14 (sCD14) und/oder pharmakologisch wirksame Fragmente des CD14 enthält.

3. Arzneimittel gemäß einem der Ansprüche 1 und/oder 2, dadurch gekennzeichnet, daß es glycosyliertes CD14 enthält.

4. Arzneimittel gemäß mindestens einem der Ansprüche 1 bis 3, dadurch gekennzeichnet, daß es trägergebundenes CD14 und/oder CD14-Derivate enthält.

5. Arzneimittel gemäß mindestens einem der Ansprüche 1 bis 4, dadurch gekennzeichnet, daß es an Immunglobuline oder Immunglobulinfraktionen gekoppeltes CD14 und/oder CD14-Derivat enthält.

6. In vitro-Verwendung von CD14 als Neutralisierungsmittel für Endotoxine.

7. Verwendung von CD14 zur Herstellung eines Arzneimittels zur Therapie oder Prophylaxe von viralen oder bakteriellen Infektionen zur Therapie von Verbrennungen und/oder zur Therapie der durch Endotoxine bedingten Erkrankungen.

8. Verwendung von CD14 zur Herstellung eines Arzneimittels zur Therapie einer Sepsis sowie des endotoxischen Schocks.

9. Verwendung von CD14 zur Herstellung eines Arzneimittels zur Behandlung von Tumoren welches in vitro hergestellte Lymphokin-aktivierte Killerzellen enthält (LAK-Therapie).

10. Verwendung von CD14 als Zusatz zu Materialien wie Kulturmedien, Zusätze, Seren, Plastikmaterialien, wie sie in klinischen und biologischen Laboratorien zur Anzüchtung und Aufbewahrung von Zellkulturen verwendet werden.

11. Verwendung von CD14 als Zusatz in Organspüllösungen, Perfusionslösungen oder sonstigen Lösungen zur Aufbewahrung von Organ- oder Gewebetransplantaten.

12. Verwendung von CD14 als Zusatz zu Lösungen oder anderen Applikationsformen von rekombinant hergestellten Arzneimitteln.

13. Verwendung gemäß mindestens einem der Ansprüche 6 bis 12, dadurch gekennzeichnet, daß als CD14 dessen pharmakologisch wirksame Fragmente eingesetzt werden.

## Claims

1. A medicament containing CD14 which has been isolated from the serum or other body fluids of humans and animals.

2. The medicament according to claim 1, characterized by containing soluble CD14 (sCD14) and/or pharmacologically effective fragments of CD14.

3. The medicament according to any of claims 1 and/or 2, characterized by containing glycosylized CD14.

4. The medicament according to at least one of claims 1 to 3, characterized by containing supported CD14 and/or CD14 derivatives.

5. The medicament according to at least one of claims 1 to 4, characterized by containing CD14 and/or a CD14 derivative coupled to immunoglobulines or immunoglobuline fractions.

6. Use of CD14 in vitro as a neutralizing agent for endotoxins.

7. Use of CD14 for the preparation of a medicament for the therapy or prophylaxis of viral or bacterial infections for the therapy of burns and/or for the therapy of diseases caused by endotoxins.

8. Use of CD14 for the preparation of a medicament for the therapy of sepsis and of endotoxic shock.

9. Use of CD14 for the preparation of a medicament for the treatment of tumors which contains lymphokin activated killer cells prepared in vitro (LAK therapy).

10. Use of CD14 as an additive for materials such as culture media, supplements, sera, plastic materials as used in clinical and biological laboratories for growing and storing cell cultures.

11. Use of CD14 as an additive for organ washing solutions, perfusion solutions or other solutions for storing organ or tissue grafts.

12. Use of CD14 as an additive for solutions or other application forms of medicaments prepared by recombinant techniques.

13. The use according to at least one of claims 6 to 12, characterized in that as said CD14, the pharmacologically effective fragments thereof are employed.

## Revendications

1. Médicament comprenant CD14 isolé du sérum ou d'autres liquides du corps de l'homme und d'animaux.

2. Médicament selon la revendication 1, caractérisé en ce qu'il comprend du CD14 soluble (sCD14) et/ou des fragments de CD14 pharmacologiquement effectives.

3. Médicament selon l'une des revendications 1 et/ou 2, caractérisé en ce qu'il comprend du CD14 glycosylé.

4. Médicament selon au moins l'une des revendications 1 à 3, caractérisé en ce qu'il comprend du CD14 et/ou des dérivés de CD14 lié ou liés à un support.

5. Médicament selon au moins l'une des revendications 1 à 4, caractérisé en ce qu'il comprend du CD14 et/ou un dérivé de CD14 couplé à des immunoglobulines ou des fractions d'immunoglobulines.

6. Utilisation du CD14 in vitro comme agent de neutralisation pour endotoxines.

7. Utilisation du CD14 pour la préparation d'un médicament pour la thérapie ou prophylaxie d'infections virales ou bactériennes pour la thérapie des brûlures et/ou pour la thérapie des maladies causées par des endotoxines.

8. Utilisation du CD14 pour la préparation d'un médicament pour la thérapie d'un état septique et du choq endotoxique.

9. Utilisation du CD14 pour la préparation d'un médicament pour le traitement des tumeurs comprenant des lymphocytes tueurs activés par la lymphokine préparés in vitro (thérapie LAK).

10. Utilisation du CD14 comme additive pour des matériaux tels que des milieux de culture, des suppléments, des sérums, des matériaux plastiques, tels que l'on utilise dans des laboratoires cliniques et biologiques pour cultiver et conserver des cultures cellulaires.

11. Utilisation du CD14 comme additive pour des solutions pour laver les organes, des solutions à perfusion ou d'autres solutions pour conserver des greffes d'organe ou de tissue.

12. Utilisation du CD14 comme additive pour des solutions ou d'autres formes d'application de médicaments préparés par des techniques recombinants.

13. Utilisation selon au moins l'une des revendications 6 à 12, caractérisée en ce qu'on utilise comme ledit CD14 ses fragments pharmacologiquement effectives.
